# NEUE EUROPÄISCHE PATENTSCHRIFT

(11) **EP 2 224 823 B2**
(45) Veröffentlichungstag und Bekanntmachung des Hinweises auf die Entscheidung über den Einspruch: **17.11.2021**
(45) Hinweis auf die Patenterteilung: 25.03.2015
(21) Anmeldenummer: 08853935.8
(22) Anmeldetag: 11.11.2008
(51) Int. Cl.: A23L 29/219, A23L 2/39, A23P 10/47, A23L 5/44, A23L 33/105, A23K 40/10, A23K 20/174, A23L 33/155

(54) **PULVERFÖRMIGE CAROTINOIDZUBEREITUNG FÜR DIE FÄRBUNG VON GETRÄNKEN**
PULVERULENT CAROTENOID PREPARATION FOR COLOURING DRINKS
PRÉPARATION PULVÉRULENTE DE CAROTÉNOÏDE POUR LA COLORATION DE BOISSONS

(30) Priorität: 29.11.2007 EP 07121904
(43) Veröffentlichungstag der Anmeldung: 08.09.2010
(73) Patentinhaber: BASF SE, 67056 Ludwigshafen am Rhein (DE)
(72) Erfinder: KÖPSEL, Christian, 69469 Weinheim (DE)
(74) Vertreter: BASF IP Association
(86) Internationale Anmeldenummer: PCT/EP2008/065285
(87) Internationale Veröffentlichungsnummer: WO 2009/068432

(56) Entgegenhaltungen:
- EP-A- 1 228 705
- EP-A2- 0 065 193
- WO-A-2007/003543
- WO-A-2007/009601
- WO-A-2007/045488
- WO-A2-2006/032399
- US-B1- 6 235 315
- "Modifizierte Stärken" In: H.-D. BELITZ; W. GROSCH: "Lehrbuch der Lebensmittelchemie", 1992, SPRINGER-VERLAG, Berlin pages 292-294,

## Beschreibung

Die vorliegende Erfindung betrifft pulverförmige Zubereitungen mindestens eines Carotinoids, ausgewählt aus der Gruppe bestehend aus β-Carotin, Astaxanthin, Canthaxanthin, Citranaxanthin, Lycopin und Lutein, ein Verfahren zur Herstellung dieser pulverförmige Zubereitungen, und die Verwendung der pulverförmigen Zubereitungen.

Die Stoffklasse der Carotinoide klassifiziert man in zwei Hauptgruppen, die Carotine und die Xanthophylle. Im Unterschied zu den Carotinen, bei denen es sich um reine Polyen-Kohlenwasserstoffe handelt, wie beispielsweise β-Carotin oder Lycopin, kommen in den Xanthophyllen noch Sauerstoff-Funktionen wie Hydroxy-, Epoxy- und/oder Carbonylgruppen vor. Typische Vertreter dieser Gruppe sind u.a. Astaxanthin, Canthaxanthin, Lutein und Zeaxanthin.

Lycopin ist der Farbstoff, der Tomaten und Hagebutten die rote Farbe verleiht. Lutein ist ein orangegelber Farbstoff, der unter anderem in den Blütenblättern der Studentenblume (Tagetes) gefunden wird, und der daraus gewonnen werden kann.

Diese sowohl synthetisch zugänglichen als auch aus natürlichen Quellen isolierbaren Polyene stellen für die Lebens- und Futtermittelindustrie sowie für den pharmazeutischen Bereich wichtige Farb- und Wirkstoffe dar und sind, wie im Falle von Astaxanthin, Wirkstoffe mit Provitamin-A Aktivität beim Lachs.

Sowohl Carotine als auch Xanthophylle sind in Wasser unlöslich, während in Fetten und Ölen eine jedoch nur geringe Löslichkeit gefunden wird. Diese begrenzte Löslichkeit sowie die hohe Oxidationsempfindlichkeit stehen einer direkten Anwendung der durch chemische Synthese erhaltenen, relativ grobkörnigen Produkte in der Einfärbung von Lebens- und Futtermitteln entgegen, da die Substanzen in grobkristalliner Form nicht lagerstabil und nur schlechte Färbungsergebnisse liefern. Diese für die praktische Verwendung der Carotinoide nachteiligen Effekte wirken sich insbesondere im wässrigen Medium aus.

Nur durch gezielt hergestellte Formulierungen, in denen die Wirkstoffe in fein verteilter Form und gegebenenfalls durch Schutzkolloide oxidationsgeschützt vorliegen, lassen sich bei der direkten Einfärbung von Lebensmitteln verbesserte Farbausbeuten erzielen. Außerdem führen diese in Futtermitteln verwendeten Formulierungen zu einer höheren Bioverfügbarkeit der Carotinoide, das heißt der Carotine oder Xanthophylle, und damit indirekt zu besseren Färbungseffekten z. B. bei der Eidotter- oder Fischpigmentierung.

Zur Verbesserung der Farbausbeuten und zur Erhöhung der Resorbierbarkeit bzw. Bioverfügbarkeit sind verschiedene Verfahren beschrieben worden, die alle das Ziel haben, die Kristallitgröße der Wirkstoffe zu verkleinern und auf einen Teilchengrößenbereich von kleiner 10 µm zu bringen.

Zahlreiche Methoden, u.a. beschrieben in Chimia 21, 329 (1967), WO 91/06292 sowie in WO 94/19411, bedienen sich dabei der Vermahlung von Carotinoiden mittels einer Kolloidmühle und erzielen damit Partikelgrößen von 2 bis 10 µm.

In WO 2007/003543 wird ein Mahlverfahren beschrieben, bei dem β-Carotin als Suspension durch Mahlen in Gegenwart von Saccharose oder Glucose und modifizierter Stärke auf eine Partikelgröße von etwa 0,6 µm zerkleinert wird und die Carotinoidhaltige Suspension anschließend in ein Trockenpulver überführt wird. Die so erhaltenen Pulver zeigen bezüglich β-Carotin eine gute Lagerstabilität in Multivitamin Tabletten.

Neben den Mahlverfahren existieren eine Reihe von kombinierten Emulgier-/Sprüh trocknungsverfahren, wie sie z. B. in DE-A-12 11 911 oder in EP-A-0 410 236 beschrieben sind.

Gemäß der europäischen Patentschrift EP-B-0 065 193 erfolgt die Herstellung von feinverteilten, pulverförmigen Carotinoidpräparaten dadurch, dass man ein Carotinoid in einem flüchtigen, mit Wasser mischbaren organischen Lösungsmittel bei erhöhten Temperaturen, gegebenenfalls unter erhöhtem Druck löst, das Carotinoid durch Mischen mit einer wässrigen Lösung eines Schutzkolloids ausfällt und anschließend sprühtrocknet.

Ein analoges Verfahren zur Herstellung von feinverteilten, pulverförmigen Carotinoidpräparaten wird in EP-A-0 937 412 unter Verwendung von mit Wasser nicht mischbaren Lösungsmitteln beschrieben.

In US 6 235 315 werden lagerstabile, pulverförmige Lycopin-Formulierungen beschrieben, die als Schutzkolloid Fischgelatine und als Weichmacher Glucose enthalten.

WO 2007/009601 offenbart ein Verfahren zur Herstellung einer pulverförmigen Zubereitung aus Carotinoiden, Octenyl_Succinat Stärke und z.B. Glucose, wobei das Carotinoid in einem Triglycerid gelöst wird, bevor es nach Vermischen mit den anderen Komponenten in ein Trockenpulver überführt wird.

Trotz der eingangs im genannten Stand der Technik beschriebenen Carotinoidformulierungen besteht seitens der Getränkeindustrie weiterhin Bedarf nach verbesserten Zubereitungen, insbesondere pulverförmigen Formulierungen von Lycopin oder Lutein, die sich leicht in Wasser, insbesondere kaltem Wasser auflösen lassen, die auch in Getränken mit hoher Wasserhärte einsetzbar sind, die eine hohe Farbintensität zeigen oder die stabile Emulsionen in den fertigen Getränken bilden, und die außerdem frei von Gelatine sind. Diese Aufgabe wird durch eine pulverförmige Zubereitung enthaltend:

| | |
|---|---|
| 5 bis 15 Gew.-% | mindestens eines Carotinoids, ausgewählt aus der Gruppe bestehend aus β-Carotin, Astaxanthin, Canthaxanthin, Citranaxanthin, Lycopin und Lutein, |
| 20 bis 55 Gew.-% | modifizierte Stärke, |
| 20 bis 55 Gew.-% | Glucose, |
| 0,7 bis 5 Gew.-% | mindestens eines Antioxidans, und |
| 0,5 bis 6,5 Gew.-% | Wasser-Restfeuchte, |

wobei sich die Gew.-%-Angaben auf das getrocknete, noch Restfeuchte enthaltende Pulver beziehen und worin
das Gewichtsverhältnis der Carotinoide zu der modifizierten Stärke 1 : 3 bis 1 : 7, bevorzugt 1 : 3 bis 1 : 5 beträgt, und wobei es sich bei der modifizierten Stärke um eine Octenyl-Succinat Stärke handelt,
und welche nach dem Verfahren von Anspruch 1 hergestellt wurde gelöst.

Die erfindungsgemäße pulverförmige Zubereitung enthält 5 bis 15 Gew.-%, insbesondere 8 bis 13 Gew.-% eines Carotinoids, ausgewählt aus der Gruppe bestehend aus β-Carotin, Astaxanthin, Canthaxanthin, Citranaxanthin, Lycopin und Lutein, bevorzugt ausgewählt aus der Gruppe bestehend aus Lycopin und Lutein. Insbesondere handelt es sich bei dem Carotinoid um Lycopin.

Weiterhin enthält die erfindungsgemäße pulverförmige Zubereitung 20 bis 55 Gew.-%, besonders bevorzugt 30 bis 50 Gew.-%, insbesondere 35 bis 45 Gew.-% modifizierte Stärke.

Unter modifizierter Stärke sind chemisch oder enzymatisch hergestellte Umwandlungsprodukte der Stärke zu verstehen. Dabei kann es sich um Stärkeether, Stärkeester oder Stärkephosphate handeln. Die erfindungsgemäße Zubereitung enthält eine Octenyl-Succinat Stärke wie beispielsweise Capsul^{®} (Natriumoctenylsuccinat Stärke) oder Purity^{®} Gum 2000 (Natriumoctenylsuccinat Stärke) der Fa. National Starch, insbesondere eine Natriumoctenylsuccinat Stärke wie Purity^{®} Gum 2000.

Die erfindungsgemäße pulverförmige Zubereitung enthält 20 bis 55 Gew.-%, besonders bevorzugt 30 bis 50 Gew.-% insbesondere 35 bis 45 Gew.-% Glucose.

Weiterhin enthält die erfindungsgemäße pulverförmige Zubereitung 0,7 bis 5 Gew.-%, besonders bevorzugt 0,8 bis 2,5 Gew.-%, ganz besonders bevorzugt 1 bis 2 Gew.-% mindestens eines Antioxidans. Beispiele für geeignete Antioxidantien sind u.a. alpha-Tocopherol, t-Butyl-hydroxy-toluol, t-Butylhydroxyanisol, Zitronensäure, Natriumcitrat, Ascorbinsäure, Natriumascorbat, Ascorbylpalmitat oder Ethoxyquin oder Mischungen davon. Bevorzugte Antioxidantien sind alpha-Tocopherol, Ascorbin-säure, Natriumascorbat, Ascorbylpalmitat oder Mischungen davon. Ganz besonders bevorzugt ist das Antioxidans alpha-Tocopherol.

Weiterhin enthält die erfindungsgemäße pulverförmige Zubereitung 0,5 bis 6,5 Gew.-% Wasser-Restfeuchte.

Zur Erhöhung der Stabilität der pulverförmigen Zubereitung gegen mikrobiellen Abbau kann es zweckmäßig sein, der Zubereitung Konservierungsmittel wie z.B. Methyl-4-hydroxybenzoat, Propyl-4-hydroxybenzoat, Sorbinsäure oder Benzoesäure oder deren Salze zuzusetzen.

Die erfindungsgemäße pulverförmige Zubereitung kann gegebenenfalls auch Emulgatoren enthalten, die in ausgesuchten Fällen bevorzugt bei der Herstellung der Dispersion eingesetzt werden. Beispiele für Emulgatoren sind Ascorbylpalmitat, Polyglycerin-Fettsäureester, wie Polyglycerin-3-polyricinoleat (PGPR 90), Sorbitan-Fettsäureester, wie Sorbitanmonostearat (span 60), PEG(20)-Sorbitolmonooleat, Propylenglykol-Fettsäureester oder Phospholipide, wie Lecithin.

Neben der Octenyl-Succinat Stärke können die erfindungsgemäßen wässrigen Suspensionen und die daraus hergestellten pulverförmigen Zubereitungen weitere Schutzkolloide enthalten. Dafür kommen beispielsweise die folgenden Stoffe in Frage:

Rinder-, Schweine- oder Fischgelatine, insbesondere sauer oder basisch abgebaute Gelatine mit Bloom-Zahlen im Bereich von 0 bis 250, ganz besonders bevorzugt Gelatine A 100, A 200, A 240, B 100 und B 200 sowie niedermolekulare, enzymatisch abgebaute Gelatinetypen mit der Bloom-Zahl 0 und Molekulargewichten von 15.000 bis 25.000 D wie zum Beispiel Collagel A und Gelitasol P (Firma Stoess, Eberbach) sowie Mischungen dieser Gelatine-Sorten.

Stärke, Dextrin, Pektin, Gummiarabicum, Ligninsulfonate, Chitosan, Polystyrolsulfonat, Alginate, Casein, Caseinat, Methylcellulose, Carboxymethylcellulose, Hydroxypropylcellulose oder Mischungen dieser Schutzkolloide.

Pflanzenproteine wie Soja-, Reis- und/oder Weizenproteine, wobei diese Pflanzenproteine teilabgebaut oder in nicht abgebauter Form vorliegen können.

In der erfindungsgemäßen pulverförmigen Zubereitung beträgt das Gewichtsverhältnis von modifizierter Stärke zu Glucose bevorzugt 4 : 1 bis 1 : 3, insbesondere 1,5 : 1 bis 1 : 1,5.

Besonders bevorzugt ist eine erfindungsgemäße pulverförmige Zubereitung wie vorangehend beschrieben, wobei der Gehalt der Carotinoide in der Zubereitung 8 bis 13 Gew.-% ist, und das Gewichtsverhältnis der Carotinoide zu modifizierter Stärke 1 : 3 bis 1 : 5 und das Gewichtsverhältnis von modifizierter Stärke zu Glucose 1,5 : 1 bis 1 : 1,5 beträgt.

In der erfindungsgemäßen pulverförmigen Zubereitung liegt das Carotinoid bevorzugt als nanopartikuläres Teilchen vor.

Als nanopartikuläre Teilchen sind solche Teilchen zu verstehen, die eine über Fraunhofer Beugung ermittelte mittlere Partikelgröße D[4,3] von 0,02 bis 100 µm, bevorzugt 0,05 bis 50 µm, besonders bevorzugt 0,05 bis 20 µm, ganz besonders bevorzugt 0,05 bis 5 µm, insbesondere 0,05 bis 1,0 µm aufweisen. Der Begriff D[4,3] bezeichnet den volumengewichteten mittleren Durchmesser (siehe Handbuch zu Malvern Mastersizer S, Malvern Instruments Ltd., UK).

Bevorzugt weisen die Carotinoidteilchen in der erfindungsgemäßen pulverförmigen Zubereitung eine mittlere Partikelgröße D[4,3] von 0,07 bis 0,7 µm auf.

Ein weiterer Gegenstand der Erfindung ist auch ein Verfahren zur Herstellung der oben beschriebenen pulverförmigen Zubereitung enthaltend:

| | |
|---|---|
| 5 bis 15 Gew.-% | mindestens eines Carotinoids, ausgewählt aus der Gruppe bestehend aus β-Carotin, Astaxanthin, Canthaxanthin, Citranaxanthin, Lycopin und Lutein, |
| 20 bis 55 Gew.-% | modifizierte Stärke, |
| 20 bis 55 Gew.-% | Glucose, |
| 0,7 bis 5 Gew.-% | mindestens eines Antioxidans, und |
| 0,5 bis 6,5 Gew.-% | Wasser-Restfeuchte, |

wobei sich die Gew.-%-Angaben auf das getrocknete, noch Restfeuchte enthaltende Pulver beziehen und worin
das Gewichtsverhältnis der Carotinoide zu der modifizierten Stärke 1 : 3 bis 1 : 7 beträgt, und wobei es sich bei der modifizierten Stärke um eine Octenyl-Succinat Stärke handelt, umfassend die Schritte
a₁) Lösen der Carotinoide in einem mit Wasser mischbaren, organischen Lösungsmittel oder in einer Mischung aus Wasser und einem mit Wasser mischbaren, organischen Lösungsmittel bei Temperaturen größer als 30°C, oder
a₂) Lösen der Carotinoide in einem mit Wasser nicht mischbaren, organischen Lösungsmittel, ausgewählt aus der Gruppe Dimethylcarbonat, Propylencarbonat, Ethylformiat, Ethylacetat, Isopropylacetat und Methyl-tert.-butylether,
b) Mischen der nach a₁) oder a₂) erhaltenen Lösung mit einer wässrigen molekulardispersen oder kolloiddispersen Lösung eines Gemisches aus Glucose und der modifizierten Stärke, wobei die hydrophobe Phase der Carotinoide als nanodisperse Phase entsteht,
c) Überführung der gebildeten Dispersion in ein Trockenpulver durch Abtrennung der Hauptmenge des Wassers und gegebenenfalls zusätzlich verwendeter Lösungsmittel und anschließender Trocknung.

Bevorzugte Ausführungsformen hinsichtlich der Komponenten Carotinoid, modifizierte Stärke, Glucose und Antioxidans und ihren Einsatzmengen finden sich in den bereits eingangs gemachten Erläuterungen.

Die in der Stufe a₁) des erfindungsgemäßen Verfahrens verwendeten wassermischbaren Lösungsmittel sind vor allem wassermischbare, thermisch stabile, flüchtige, nur Kohlenstoff, Wasserstoff und Sauerstoff enthaltenene Lösungsmittel wie Alkohole, Ether, Ester, Ketone oder Acetale. Zweckmäßig verwendet man solche Lösungsmittel, die mindestens zu 10% wassermischbar sind, einen Siedepunkt unter 200°C aufweisen und/oder weniger als 10 Kohlenstoffatome haben. Besonders bevorzugt werden Methanol, Ethanol, n-Propanol, Isopropanol, 1,2-Butandiol-1-methylether (1-Methoxy-butanol-2), 1,2-Propandiol-1-n-propylether (1-Propoxy-propanol-2), Tetrahydrofuran oder Aceton verwendet.

In dem erfindungsgemäßen Verfahren wird bevorzugt Verfahrensschritt a₁) ausgeführt, wobei das Carotinoid in einem mit Wasser mischbaren, organischen Lösungsmittel oder in einer Mischung aus Wasser und einem mit Wasser mischbaren, organischen Lösungsmittel bei Temperaturen größer als 30°C, vorzugsweise zwischen 50°C und 240°C, insbesondere 100°C bis 200°C, besonders bevorzugt 140°C bis 180°C, gegebenenfalls unter Druck, gelöst wird.

Da die Einwirkung hoher Temperaturen unter Umständen den gewünschten hohen alltrans Isomerenanteil des Carotinoids, insbesondere von Lycopin oder Lutein herabsetzen kann, löst man das/die Carotinoid(e) möglichst rasch, beispielsweise im Sekundenbereich, z. B. in 0,1 bis 10 Sekunden, besonders bevorzugt in weniger als 1 Sekunde. Zur raschen Herstellung der molekulardispersen Lösung kann die Anwendung von erhöhtem Druck, z. B. im Bereich von 20 bar bis 80 bar, vorzugsweise 30 bis 60 bar, vorteilhaft sein.

Die so erhaltene molekulardisperse Lösung versetzt man anschließend in Verfahrensschritt b) direkt mit der gegebenenfalls gekühlten wässrigen molekulardispersen oder kolloiddispersen Lösung des Gemisches aus Glucose und der modifizierten Stärke, wobei die hydrophobe Phase der Carotinoide als nanodisperse Phase entsteht. Bevorzugt stellt sich in Verfahrensschritt b) eine Mischungstemperatur von etwa 35°C bis 80°C ein.

Dabei wird die Lösungsmittelkomponente aus Verfahrensschritt a₁) in die wässrige Phase überführt und die hydrophobe Phase des/der Carotinoid(e) entsteht als nanodisperse Phase.

Hinsichtlich einer näheren Verfahrens- und Apparatebeschreibung zur oben genannten Dispergierung wird an dieser Stelle auf EP-B-0 065 193 Bezug genommen.

In dem erfindungsgemäßen Verfahren wird in Verfahrensschritt c) die gebildete Dispersion durch Abtrennung der Hauptmenge des Wassers und gegebenenfalls zusätzlich verwendeter Lösungsmittel und anschließender Trocknung in ein Trockenpulver überführt.

Die Überführung in ein Trockenpulver kann dabei u.a. durch Sprühtrocknung, Sprühkühlung, modifizierte Sprühtrocknung, Gefriertrocknung oder Trocknung im Wirbelbett, gegebenenfalls auch in Gegenwart eines Überzugsmaterials erfolgen. Als Überzugsmittel eignen sich u.a. Maisstärke, Kieselsäure oder auch Tricalciumphosphat.

Bevorzugt wird in Verfahrensschritt c) die gebildete Dispersion durch destillative Abtrennung der Hauptmenge des Wassers und eventuell zusätzlich vorhandener Lösungsmittel auf eine Feststoffkonzentration von etwa 20 bis 35 Gew.-% aufkonzentriert und anschließend diese aufkonzentrierte Dispersion in einem Sprühtrockner in ein Trockenpulver überführt.

Besonders bevorzugt wird in Verfahrensschritt c) des erfindungsgemäßen Verfahrens die Trocknung in einem Sprühtrockner mit integriertem und/oder nachgeschaltetem, externen Wirbelbett durchgeführt. Dabei wird bevorzugt eine pulverförmige Zubereitung mit agglomerierten Partikeln gebildet. Die erhaltenen agglomerierten Partikel weisen bevorzugt je nach eingesetzter Apparatur die Struktur einer Zwiebel, einer Himbeere, einer kompakten Traube oder einer lockeren Traube auf. Diese Agglomerat-Strukturtypen, die resultierenden Pulvereigenschaften sowie Agglomerationsverfahren werden beispielsweise von Ejnar Refstrup, "Recent Advances in Agglomeration during Spray Drying", in der "Zeitschrift für Lebensmitteltechnologie", ZFL 43 (1992), 10, Seite 576 bis 582 beschrieben. Die agglomerierten Partikel der erfindungsgemäßen pulverförmige Zubereitung weisen besonders bevorzugt die Struktur einer Himbeere oder einer kompakten oder lockere Traube, insbesondere einer kompakten oder lockere Traube auf.

Die erfindungsgemäßen Trockenpulver zeichnen sich u.a. dadurch aus, dass sie sich in wässrigen Systemen unter Erzielung einer gleichmäßigen Feinverteilung des Wirkstoffes im Korngrößenbereich kleiner 1 µm problemlos wieder redispergieren lassen. Dabei ist festzustellen, dass sich die erfindungsgemäßen Trockenpulver sehr schnell in kaltem Wasser redispergieren lassen und über lange Zeit stabile Dispersionen mit hoher Farbstärke bilden.

Die erfindungsgemäße pulverförmige Zubereitung eignet sich u.a. als Zusatzstoff zu Lebensmittelzubereitungen, beispielsweise zur Färbung von Lebensmitteln wie Getränken, als Mittel für die Herstellung pharmazeutischer und kosmetischer Zubereitungen sowie für die Herstellung von Nahrungsergänzungspräparaten, beispielsweise von Multivitaminpräparaten im Human- und Tierbereich. Bevorzugt eignet sich die pulverförmige Zubereitung als Zusatz zu Getränken.

Ein weiter Gegenstand der vorliegenden Erfindung ist daher auch die Verwendung der oben beschriebenen, erfindungsgemäßen pulverförmigen Zubereitung als Zusatz zu Tierfuttermitteln, Lebensmitteln, Nahrungsergänzungsmitteln oder pharmazeutischen Mitteln, insbesondere als Zusatz zu Getränken.

Ebenfalls Gegenstand der vorliegenden Erfindung sind Tierfuttermittel, Lebensmittel, Nahrungsergänzungsmittel oder pharmazeutische Mittel, insbesondere ein Getränk, welches die erfindungsgemäße, pulverförmige Zubereitung enthält.

Die Erfindung wird durch folgende, die Erfindung jedoch nicht einschränkende Beispiele erläutert:

### Beispiele

### Beispiel 1

10 g kristallines Lycopin wurden in einer Lösung aus 1,7 g α-Tocopherol und 130 g Isopropanol suspendiert. Diese Suspension wurde mit einer Förderrate von 1,5 kg/h und 50 bar Systemdruck mit 460 g heißem Isopropanol (Förderrate 2,6 kg/h) zu einer Lösetemperatur von 170°C gemischt. Diese Lösung wurde in einer Mischkammer turbulent mit einer Lösung von 70,4 g Purity Gum 2000 (National Starch) und 80,4 g Glucose in 6450 g Wasser gemischt. Aus dieser Lycopindispersion wurde anschließend das Isopropanol mittels eines Vakuumverdampfers entfernt, und die Dispersion durch Sprühtrocknung in ein Pulver überführt. Der Gehalt an Lycopin in dem Pulver betrug 10,9 %, und die mittlere Teilchengröße der Lycopinteilchen betrug 307 nm.

## Patentansprüche

1. Verfahren zur Herstellung einer pulverförmige Zubereitung enthaltend:
| | |
|---|---|
| 5-15 Gew.-% | mindestens eines Carotinoids, ausgewählt aus der Gruppe bestehend aus β-Carotin, Astaxanthin, Canthaxanthin, Citranaxanthin, Lycopin und Lutein, |
| 20 bis 55 Gew.-% | modifizierte Stärke, |
| 20 bis 55 Gew.-% | Glucose, |
| 0,7 bis 5 Gew.-% | mindestens eines Antioxidans, und |
| 0,5 bis 6,5 Gew.-% | Wasser-Restfeuchte, |
wobei sich die Gew.-%-Angaben auf das getrocknete, noch Restfeuchte enthaltende Pulver beziehen und worin
das Gewichtsverhältnis der Carotinoide zu der modifizierten Stärke 1 : 3 bis 1 : 7 beträgt, und wobei
es sich bei der modifizierten Stärke um eine Octenyl-Succinat Stärke handelt,
umfassend die Schritte
a₁) Lösen der Carotinoide in einem mit Wasser mischbaren, organischen Lösungsmittel oder in einer Mischung aus Wasser und einem mit Wasser mischbaren, organischen Lösungsmittel bei Temperaturen größer als 30°C, oder
a₂) Lösen der Carotinoide in einem mit Wasser nicht mischbaren, organischen Lösungsmittel ausgewählt aus der Gruppe Dimethylcarbonat, Propylencarbonat, Ethylformiat, Ethylacetat, Isopropyl-acetat und Methyl-tert.-butylether,
b) Mischen der nach a₁) oder a₂) erhaltenen Lösung mit einer wässrigen molekulardispersen oder kolloiddispersen Lösung eines Gemisches aus Glucose und der modifizierten Stärke, wobei die hydrophobe Phase der Carotinoide als nanodisperse Phase entsteht,
c) Überführung der gebildeten Dispersion in ein Trockenpulver durch Abtrennung der Hauptmenge des Wassers und gegebenenfalls zusätzlich verwendeter Lösungsmittel und anschließender Trocknung.

2. Verfahren nach Anspruch 1, wobei in Schritt c) die gebildete Dispersion durch destillative Abtrennung der Hauptmenge des Wassers und eventuell zusätzlich vorhandener Lösungsmittel auf eine Feststoffkonzentration von etwa 20 bis 35 Gew.-% aufkonzentriert wird und anschließend diese aufkonzentrierte Dispersion in einem Sprühtrockner in ein Trockenpulver überführt wird.

3. Verfahren nach Anspruch 2 oder 3, wobei die Trocknung in Schritt (c) in einem Sprühtrockner mit integriertem und/oder nachgeschaltetem, externen Wirbelbett durchgeführt wird.

4. Verfahren nach mindestens einem der Ansprüche 1 bis 3, wobei es sich bei dem Carotinoid um Lycopin handelt.

5. Verfahren nach mindestens einem der Ansprüche 1 bis 4, wobei das Antioxidans alpha-Tocopherol ist.

6. Verfahren nach mindestens einem der Ansprüche 1 bis 6, wobei das Gewichtsverhältnis von modifizierter Stärke zu Glucose 1,5 : 1 bis 1 : 1,5 beträgt.

7. Verfahren nach mindestens einem der Ansprüche 1 bis 5, wobei der Gehalt der Carotinoide in der Zubereitung 8 bis 13 Gew.-% ist, und das Gewichtsverhältnis der Carotinoide zu modifizierter Stärke 1 : 3 bis 1 : 5 und das Gewichtsverhältnis von modifizierter Stärke zu Glucose 1,5 : 1 bis 1 : 1,5 beträgt.

8. Pulverförmige Zubereitung hergestellt nach einem Verfahren gemäß mindestens einem der Ansprüche 1 bis 7.

9. Verwendung der pulverförmigen Zubereitung gemäß Anspruch 8 als Zusatz zu Tierfuttermitteln, Lebensmitteln, Nahrungsergänzungsmitteln oder pharmazeutischen Mitteln.

10. Verwendung der pulverförmigen Zubereitung gemäß Anspruch 8, als Zusatz zu Getränken.

11. Tierfuttermittel, Lebensmittel, Nahrungsergänzungsmittel oder pharmazeutische Mittel, umfassend die pulverförmige Zubereitung gemäß Anspruch 8.

12. Getränk, umfassend die pulverförmige Zubereitung gemäß Anspruch 8.

## Claims

1. A process for producing a pulverulent composition comprising:
| | |
|---|---|
| 5-15% by weight | of at least one carotenoid selected from the group consisting of β-carotene, astaxanthin, canthaxanthin, citranaxanthin, lycopene and lutein, |
| 20 to 55% by weight | of modified starch, |
| 20 to 55% by weight | of glucose, |
| 0.7 to 5% by weight | of at least one antioxidant, and |
| 0.5 to 6.5% by weight | of water-residual moisture, |
wherein the percentages by weight relate to the dried powder still comprising residual moisture and where
the weight ratio of the carotenoids to the modified starch is 1:3 to 1:7, and wherein the modified starch is an octenyl succinate starch,
comprising the steps
a₁) dissolving the carotenoids in a water-miscible organic solvent or in a mixture of water and a water-miscible organic solvent at temperatures above 30°C, or
a₂) dissolving the carotenoids in a waterimmiscible organic solvent selected from the group consisting of dimethyl carbonate, propylene carbonate, ethyl formate, ethyl acetate, isopropyl acetate and methyl tert-butyl ether,
b) mixing the solution obtained according to a₁) or a₂) with an aqueous molecularly disperse or colloidally disperse solution of a mixture of glucose and the modified starch, wherein the hydrophobic phase of the carotenoids is formed as nanodisperse phase,
c) converting the dispersion formed into a dry powder by separating off the majority of the water and, optionally, solvents additionally used, and subsequent drying.

2. The process according to claim 1, wherein, in step c), the dispersion formed is concentrated to a solids concentration of about 20 to 35% by weight by separating off the majority of the water and any solvents additionally present by distillation and subsequently converting this concentrated dispersion into a dry powder in a spray drier.

3. The process according to claim 2 or 3, wherein the drying in step c) is carried out in a spray drier having an integrated and/or downstream external fluidized bed.

4. The process according to at least one of claims 1 to 3, wherein the carotenoid is lycopene.

5. The process according to at least one of claims 1 to 4, wherein the antioxidant is alpha-tocopherol.

6. The process according to at least one of claims 1 to 6, wherein the weight ratio of modified starch to glucose is 1.5:1 to 1:1.5.

7. The process according to at least one of claims 1 to 5, wherein the content of carotenoids in the composition is 8 to 13% by weight, and the weight ratio of the carotenoids to modified starch is 1:3 to 1:5 and the weight ratio of modified starch to glucose is 1.5:1 to 1:1.5.

8. A pulverulent composition produced by a process according to at least one of claims 1 to 7.

9. The use of the pulverulent composition according to claim 8 as additive to animal feeds, foods, food supplements or pharmaceutical compositions.

10. The use of the pulverulent composition according to claim 8 as additive to drinks.

11. An animal feed, food, food supplement or pharmaceutical composition comprising the pulverulent composition according to claim 8.

12. A drink comprising the pulverulent composition according to claim 8.

## Revendications

1. Procédé pour la production d'une préparation pulvérulente contenant :
| | |
|---|---|
| 5 à 15 % en poids | d'au moins un caroténoïde, choisi dans le groupe constitué par le β-carotène, l'astaxanthine, la canthaxanthine, la citranaxanthine, le lycopène et la lutéine, |
| 20 à 55 % en poids | d'amidon modifié, |
| 20 à 55 % en poids | de glucose, |
| 0,7 à 5 % en poids | d'au moins un antioxydant et |
| 0,5 à 6,5 % en poids | d'eau-humidité résiduelle, |
les données en % en poids se rapportant à la poudre séchée, contenant encore de l'humidité résiduelle et dans lequel
le rapport pondéral des caroténoïdes à l'amidon modifié vaut de 1 : 3 à 1 : 7, et
l'amidon modifié consistant en un succinate d'octénylamidon,
comprenant les étapes
a₁) dissolution des caroténoïdes dans un solvant organique miscible à l'eau ou dans un mélange d'eau et d'un solvant organique miscible à l'eau, à des températures supérieures à 30 °C, ou
a₂) dissolution des caroténoïdes dans un solvant organique non miscible à l'eau, choisi dans le groupe constitué par le carbonate de diméthyle, le carbonate de propylène, le formiate d'éthyle, l'acétate d'éthyle, l'acétate d'isopropyle et l'éther de méthyle et de tert-butyle,
b) mélange de la solution obtenue selon a₁) ou a₂) avec une solution aqueuse en dispersion moléculaire ou dispersion colloïdale d'un mélange de glucose et de l'amidon modifié, la phase hydrophobe des caroténoïdes étant formée en tant que phase nanodipersée,
c) conversion de la dispersion formée en une poudre sèche par séparation de la majeure partie de l'eau et des solvants éventuellement utilisés en plus, et séchage subséquent.

2. Procédé selon la revendication 1, dans lequel dans l'étape c) on concentre la dispersion formée, par séparation par distillation de la majeure partie de l'eau et des solvants éventuellement présents en plus, jusqu'à une concentration de matière solide d'environ 20 à 35 % en poids et ensuite on convertit cette dispersion concentrée, dans un sécheur par atomisation, en une poudre sèche.

3. Procédé selon la revendication 2 ou 3, dans lequel dans l'étape (c) on effectue le séchage dans un sécheur par atomisation à lit tourbillonnaire intégré et/ou externe raccordé en aval.

4. Procédé selon au moins l'une quelconque des revendications 1 à 3, dans lequel pour ce qui est du caroténoïde il s'agit de lycopène.

5. Procédé selon au moins l'une quelconque des revendications 1 à 4, dans lequel l'antioxydant est l'alpha-tocophérol.

6. Procédé selon au moins l'une quelconque des revendications 1 à 6, dans lequel le rapport pondéral de l'amidon modifié au glucose vaut de 1,5 : 1 à 1 : 1,5.

7. Procédé selon au moins l'une quelconque des revendications 1 à 5, dans lequel la teneur en caroténoïdes dans la préparation vaut 8 à 13 % en poids, et le rapport pondéral des caroténoïdes à l'amidon modifié vaut 1 : 3 à 1 : 5 et le rapport pondéral de l'amidon modifié au glucose vaut 1,5 : 1 à 1 : 1,5.

8. Préparation pulvérulente, produite conformément à un procédé selon au moins l'une quelconque des revendications 1 à 7.

9. Utilisation de la préparation pulvérulente selon la revendication 8, comme additif à des aliments pour animaux, des produits alimentaires, des compléments nutritionnels ou des produits pharmaceutiques.

10. Utilisation de la préparation pulvérulente selon la revendication 8, comme additif à des boissons.

11. Aliments pour animaux, produits alimentaires, compléments nutritionnels ou produits pharmaceutiques, comprenant la préparation pulvérulente selon la revendication 8.

12. Boisson, comprenant la préparation pulvérulente selon la revendication 8.
